# EUROPEAN PATENT APPLICATION

(11) **EP 4 772 206 A1**
(43) Date of publication of application: **08.07.2026**
(21) Application number: 24872239.9
(22) Date of filing: 25.09.2024
(51) Int. Cl.: A61M 1/18

(54) **ARTIFICIAL LUNG**

(30) Priority: 27.09.2023 JP 2023164700
(71) Applicant: Terumo Kabushiki Kaisha, Tokyo 151-0072 (JP)
(72) Inventor: SUZUKI, Ayato, Ashigarakami-gun, Kanagawa 259-0151 (JP); MORI, Takehisa, Ashigarakami-gun, Kanagawa 259-0151 (JP)
(74) Representative: KIPA AB
(86) International application number: PCT/JP2024/034077
(87) International publication number: WO 2025/070453

(57) **Abstract**

To suppress stagnation portion of blood flowing between a housing (10) and a hollow fiber membrane layer (30) and improve antithrombogenicity, an oxygenator (100) includes a housing (10) having a cylindrical shape and including a blood inflow port (14) and a blood outflow port (15), and a hollow fiber membrane layer (30) having a stacked structure obtained by winding hollow fiber membranes (31) accommodated in the housing (10), the oxygenator in which blood flowing in from the blood inflow port (12) flows from one end toward the other end in a longitudinal direction of the housing (10) and flows out from the blood outflow port, in which, in the hollow fiber membrane layer (30), a winding interval (P1) of the hollow fiber membranes wound in an outermost layer in the stacked structure is wider than a winding interval (P2) of the hollow fiber membranes in other layers.

## Description

### Technical Field

The present invention relates to an oxygenator.

### Background Art

Conventionally, an extracorporeal circulation system that assists circulation of blood and respiration of a patient has been widely used in order to temporarily maintain life at the time of open heart surgery of a heart disease or in a rapidly progressing circulation failure or cardiopulmonary arrest state.

The extracorporeal circulation system includes an oxygenator that is incorporated in an extracorporeal circulation circuit including a blood removal path, a blood supply path and the like and exchanges gas with blood, and a pump that is incorporated in the extracorporeal circulation circuit and sends blood to the oxygenator.

In the extracorporeal circulation system, the oxygenator performs gas exchange (gives oxygen to blood and removes carbon dioxide) on the removed blood by a hollow fiber membrane layer disposed in the oxygenator, and sends the blood to the blood supply path (for example, refer to Patent Literature 1).

### Citation List

### Patent Literature

Patent Literature 1: German Patent Application Publication No. 10341221

### Summary of Invention

### Technical Problem

In a case where an extracorporeal circulation system is used for a long period of time, there is a problem that thrombus occurs in the oxygenator. In the oxygenator, a hollow fiber membrane layer is accommodated in a cylindrical housing, and a blood flow stagnates due to winding disturbance or the like of hollow fiber membranes in the hollow fiber membrane layer, so that a thrombus may be formed between the hollow fiber membrane layer and the housing and the like.

The present invention has been made in view of the above problems, and an object thereof is to provide an oxygenator that suppresses a stagnation portion of blood flowing between a housing and a hollow fiber membrane layer and improves antithrombogenicity.

### Solution to Problem

The above object of the present invention is achieved by the following means (1) to (5).
(1) An oxygenator including a housing having a cylindrical shape and including a blood inflow port and a blood outflow port, and a hollow fiber membrane layer having a stacked structure obtained by winding hollow fiber membranes accommodated in the housing, the oxygenator in which blood flowing in from the blood inflow port flows from one end toward the other end in a longitudinal direction of the housing and flows out from the blood outflow port, in which, in the hollow fiber membrane layer, a winding interval of the hollow fiber membranes wound in an outermost layer in the stacked structure is wider than a winding interval of the hollow fiber membranes in other layers.
(2) The oxygenator according to (1) described above, in which, in the hollow fiber membrane layer, the hollow fiber membranes are sparsely wound only in the outermost layer in the stacked structure as compared with the other layers.
(3) The oxygenator according to (1) or (2) described above, in which, in the hollow fiber membrane layer, the number of windings in the outermost layer in the stacked structure is smaller than the number of windings in the other layers.
(4) The oxygenator according to any one of (1) to (3), in which, in the hollow fiber membrane layer, the winding interval of the hollow fiber membranes in the stacked structure is discontinuous, and an interval difference between the winding interval of the hollow fiber membranes in the outermost layer and the winding interval of the hollow fiber membranes in the other layers is larger than the interval difference between the winding intervals between any other layers.
(5) The oxygenator according to any one of (1) to (4) described above, in which, in the hollow fiber membrane layer, a winding interval of an outermost layer in the stacked structure is 1.5 mm or larger.

### Advantageous Effects of Invention

According to an oxygenator of the present embodiment, in a device configuration in which blood flows from one end toward the other end in a longitudinal direction of a housing from a blood inflow port to a blood outflow port, an interval of hollow fiber membranes wound in an outermost layer of a hollow fiber membrane layer is widened as compared to that in other layers so as to make a circulation direction of the blood flowing through the outermost layer of the hollow fiber membrane layer a flow from one end to the other end in the longitudinal direction of the housing. As a result, in the hollow fiber membrane layer, a circulation path having the same width as a winding interval in which blood can circulate is formed in the outermost layer, and the flow of the blood flowing between the inner peripheral surface of the housing and the hollow fiber membrane layer is defined to be directed from one end toward the other end in the longitudinal direction of the housing, so that the flow velocity of the blood flowing through the outermost layer of the hollow fiber membrane layer increases. Therefore, by increasing the flow velocity of the blood flowing through the outermost layer of the hollow fiber membrane layer, the oxygenator can suppress the formation of the stagnation portion of the blood in the outermost layer of the hollow fiber membrane layer to enhance antithrombogenicity.

### Brief Description of Drawings

Fig. 1 is a longitudinal sectional view of an oxygenator of a first embodiment according to the present embodiment.

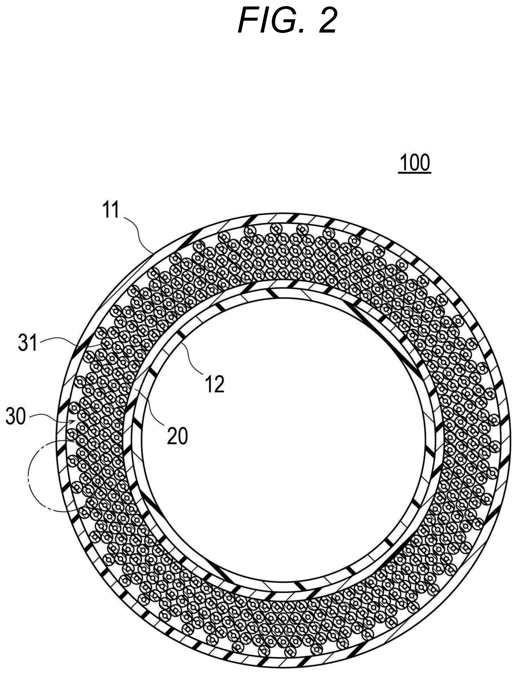

Fig. 2 is a transverse sectional view of the oxygenator of the first embodiment according to the present embodiment.
Fig. 3 is a partial transverse sectional view of a hollow fiber membrane layer of the oxygenator of the first embodiment according to the present embodiment.
Fig. 4 is a diagram of the periphery of an outermost layer in the hollow fiber membrane layer of the oxygenator of the first embodiment according to the present embodiment.
Fig. 5 is a longitudinal sectional view of an oxygenator of a second embodiment according to the present embodiment.

### Description of Embodiments

Hereinafter, a mode for carrying out the present invention will be described in detail with reference to the drawings. Embodiments herein described are illustrated to embody the technical idea of the present invention and do not limit the present invention. Other embodiments, examples, technical operations and the like that could be conceived by those skilled in the art without departing from the gist of the present invention are all included in the scope and gist of the present invention and included in the invention recited in claims and the scope of equivalents thereof.

Furthermore, for the purpose of illustration and for ease of comprehension, the scale, aspect ratio, shape and the like in the drawings attached to the present specification may be changed from actual ones as appropriate and illustrated schematically; however, the drawings are examples and do not limit the interpretation of the present invention.

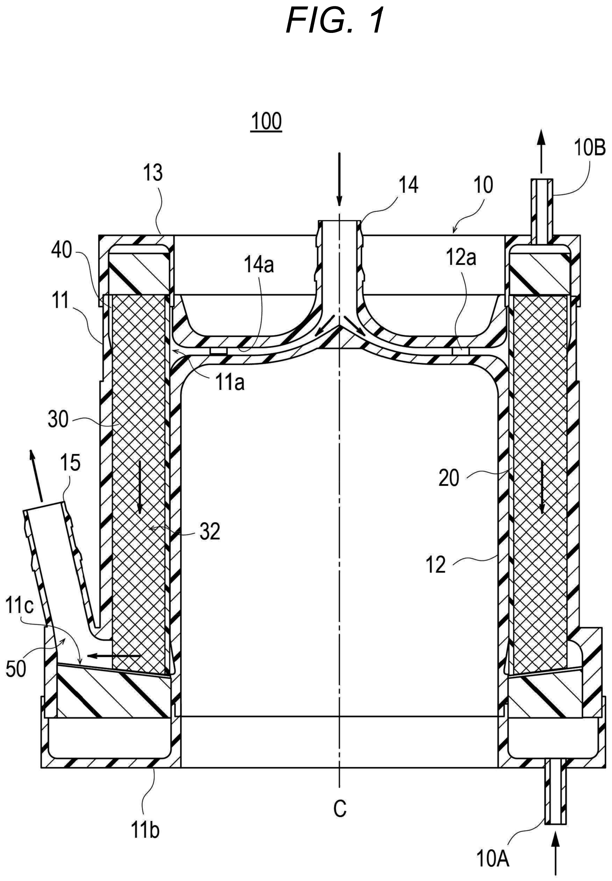

In the present specification, the following directions are defined for convenience of description. In Fig. 1, a "longitudinal direction" is a direction along a central axis C in a longitudinal direction of an oxygenator 100. A "radial direction" is a direction away from or approaching the central axis C in an axial orthogonal cross-section (transverse section) with the central axis C of the oxygenator 100 as a reference axis. A "circumferential direction" is a rotational direction with the central axis C of the oxygenator 100 as a reference axis. The central axis C substantially coincides with central axes of an outer cylinder 11 and an inner cylinder 12 forming a housing 10.

The inventor of the present application has started development of a novel oxygenator in order to solve the problem of thrombus formation due to blood stagnation, which is a problem of the conventional device. The inventor of the present application has fundamentally reviewed a blood flow path and a circulation direction of blood in the oxygenator, and has found that partial blood flow stagnation can be suppressed by allowing the blood that flows in from a blood inflow port to circulate from one end toward the other end in a longitudinal direction of a housing and flow out from a blood outflow port.

In the oxygenator including a novel blood flow path, blood flow stagnation in an outermost layer of the hollow fiber membrane layer is eliminated and antithrombogenicity is obtained, but on the other hand, a new problem has been found that blood stagnation may newly occur in a portion where the housing and the hollow fiber membrane layer are in contact with each other.

The inventor of the present application has further studied this new problem and found that the above problem can be solved by adopting a structure in which blood flowing between the housing and an outermost layer and an innermost layer of the hollow fiber membrane layer has a flow from one end toward the other end in a longitudinal direction of the housing, thereby increasing a flow velocity of the blood in the outermost layer of the hollow fiber membrane layer. The present invention is a novel oxygenator developed based on the above findings.

Note that, in the following description, ordinal numbers such as "first" and "second" are used for convenience and do not define any order unless otherwise specified.

### [Device Configuration]

An oxygenator 100 according to the present invention will be described.

The oxygenator 100 is incorporated in an extracorporeal circulation circuit and exchanges gas with blood. The oxygenator 100 includes a membrane oxygenator that exchanges gas with the blood by a hollow fiber membrane 31.

As illustrated in Fig. 1, the oxygenator 100 includes a cylindrical housing 10, a core 20 accommodated in an accommodation space 11a of the housing 10, a hollow fiber membrane layer 30 formed by a bundle of the hollow fiber membranes 31 wound around the core 20 to be stacked, a blood chamber 40 formed between the housing 10 and the hollow fiber membrane layer 30, and a blood flow path 50 serving as a flow path of the blood in the housing 10. Note that, an outer peripheral surface of the core 20 and an inner peripheral surface of the hollow fiber membrane layer 30 are partially in contact with each other. In other words, the blood chamber 40 is formed by a void in a portion in which the outer peripheral surface of the core 20 and the inner peripheral surface of the hollow fiber membrane layer 30 are not in contact with each other.

### <Housing>

The housing 10 is a casing that forms a main body of the oxygenator 100. As illustrated in Fig. 1, the housing 10 includes a cylindrical outer cylinder 11 extending in a longitudinal direction (vertical direction in Fig. 1), an inner cylinder 12 accommodated in the outer cylinder 11, a lid 13 airtightly coupled to outer peripheral ends of the outer cylinder 11 and the inner cylinder 12, a blood inflow port 14 for allowing the blood to flow into the housing 10, and a blood outflow port 15 for allowing the blood circulating in the housing 10 to flow out.

As illustrated in Fig. 1, the outer cylinder 11 has a cylindrical shape, and includes the accommodation space 11a for accommodating the core 20 and the hollow fiber membrane layer 30 therein. The blood outflow port 15 is provided at a proximal end of the outer cylinder 11 so as to extend in a direction intersecting the longitudinal direction of the outer cylinder 11.

As illustrated in Fig. 1, the inner cylinder 12 has a cylindrical shape having a diameter smaller than that of the outer cylinder 11. The inner cylinder 12 functions as an annular inner peripheral portion of the housing 10. On a distal end side of the inner cylinder 12, a plurality of protrusions 12a for positioning when arranging the blood inflow port 14 and providing a gap serving as a guide portion 14a is arranged at predetermined intervals in a circumferential direction.

The lid 13 is a bottomed annular member having a concave cross-section, and airtightly closes a distal end in the longitudinal direction of the outer cylinder 11 in a state in which the inner cylinder 12, the blood inflow port 14, the core 20, and the hollow fiber membrane layer 30 are accommodated in the outer cylinder 11.

An outer bottom 11b of the oxygenator 100 is provided with a gas inflow port 10A that allows gas for the gas exchange of the blood to flow into the housing 10. The lid 13 is provided with a gas outflow port 10B that allows gas after the gas exchange to flow out. From the viewpoint of improving efficiency of the gas exchange in the blood, the gas inflow port 10A is preferably disposed at a position (proximal end side of the outer cylinder 11 on a downstream side in the circulation direction of the blood) at which the gas can flow into the blood chamber 40 opposed to the circulation direction of the blood circulating in the accommodation space 11a (blood chamber 40). The gas outflow port 10B is preferably disposed at a position substantially opposite to the gas inflow port 10A in the longitudinal direction (on the distal end side of the outer cylinder 11 on an upstream side in the circulation direction of the blood). Note that, the arranging positions of the gas inflow port 10A and the gas outflow port 10B are not limited to the positions illustrated in Fig. 1 as long as they are positions at which gas exchange of blood is possible in the accommodation space.

The blood inflow port 14 is attachable to and detachable from the housing 10, and is disposed on the distal end side of the inner cylinder 12 of the housing 10. The blood inflow port 14 is connected to a blood removal path in the extracorporeal circulation circuit of the extracorporeal circulation system, and allows blood to flow into the housing 10. A distal end opening serving as an inflow port of the blood inflow port 14 is formed to face the distal end side in the longitudinal direction of the housing 10. The blood flowing in from the blood inflow port 14 flows into the blood chamber 40 through the guide portion 14a formed between the same and the distal end side of the inner cylinder 12.

The blood outflow port 15 extends in a direction intersecting the circulation direction of the blood flowing in the housing 10 (longitudinal direction of the housing 10) and is disposed on an outer peripheral portion of the outer cylinder 11. The blood outflow port 15 is connected to a blood supply path in the extracorporeal circulation circuit of the extracorporeal circulation system, and causes the blood circulating in the housing 10 to flow out to the blood supply path. In a mode illustrated in Fig. 1, a distal end opening of the blood outflow port 15 is formed to face the distal end side of the housing 10, similarly to a distal end opening serving as an outflow port of the blood outflow port 15. However, the direction of the distal opening of the blood outflow port 15 is not limited thereto.

The housing 10 is preferably transparent to the extent that the blood flow therein is visible. Note that, the term "transparent" in the present specification includes colorless transparent, colored transparent, and translucent.

A material forming the housing 10 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

### <Core>

The core 20 has a cylindrical shape, and is accommodated in the housing 10 in a state in which the hollow fiber membrane 31 is wound and the hollow fiber membrane layer 30 is included. The core 20 is attached to cover the inner cylinder 12.

The material forming the core 20 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

### <Hollow Fiber Membrane Layer>

As illustrated in Fig. 1, the hollow fiber membrane layer 30 is formed in a state in which the hollow fiber membranes 31 are wound around the core 20 and stacked in multiple layers.

As illustrated in Fig. 1, the hollow fiber membrane layer 30 includes a gas exchange unit 32.

The gas exchange unit 32 is formed of a bundle of the hollow fiber membranes 31. In the gas exchange unit 32, oxygen that circulates in the gas exchange unit 32 is diffused to a blood side when passing through the hollow fiber membrane 31. Carbon dioxide in the blood that circulates in the gas exchange unit 32 is discharged into a lumen of the hollow fiber membrane 31. As a result, in the gas exchange unit 32, gas exchange of oxygen and carbon dioxide is performed with the blood via the hollow fiber membrane 31.

Oxygen that flows in from the gas inflow port 10A is subjected to the gas exchange with carbon dioxide in the blood in the gas exchange unit 32, and carbon dioxide subjected to the gas exchange is discharged out of the oxygenator 100 through the gas outflow port 10B.

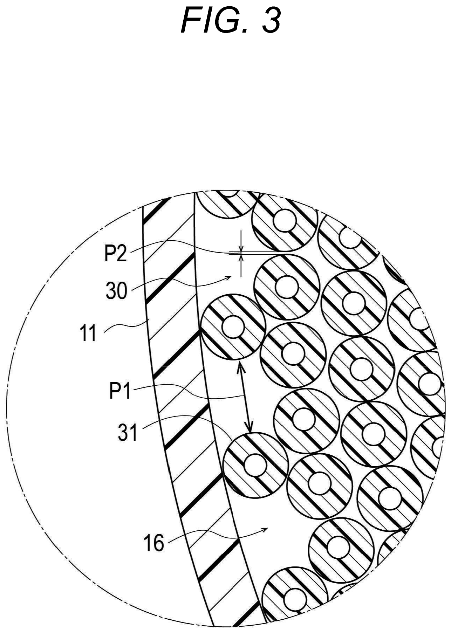

As illustrated in Fig. 3, in the hollow fiber membrane layer 30, a winding interval (corresponding to a separation distance between the hollow fiber membranes 31 adjacent in the circumferential direction) that is a gap between the hollow fiber membranes 31 wound around the core 20 is different between a winding interval P1 of the outermost layer and a winding interval P2 of the other layers in a stacked structure. Specifically, in the hollow fiber membrane layer 30, in the stacked structure, the winding interval P1 of the hollow fiber membranes 31 in the outermost layer is wider than the winding interval P2 of the hollow fiber membranes 31 in the other layers. In other words, in the hollow fiber membrane layer 30, the hollow fiber membranes 31 are sparsely wound only in the outermost layer as compared to the other layers in the stacked structure. In the hollow fiber membrane layer 30, the winding interval of the hollow fiber membranes 31 in the stacked structure is discontinuous, and an interval difference between the winding interval P1 of the hollow fiber membranes 31 in the outermost layer and the winding interval P2 of the hollow fiber membranes 31 in the other layers can be said to be larger than the interval difference between the winding intervals P2 between any other layers.

In the hollow fiber membrane layer 30, the winding interval P1 of the outermost layer is made wider than the winding interval P2 of the other layers, so that the number of windings of the hollow fiber membranes 31 in the outermost layer in the stacked structure can be made smaller than the number of windings of the hollow fiber membranes 31 in the other layers.

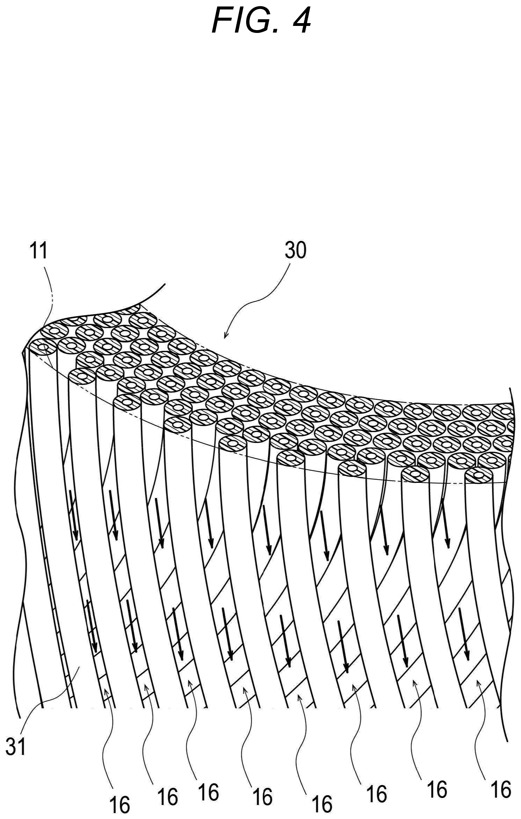

In the hollow fiber membrane layer 30, as illustrated in Fig. 4, by making the winding interval P1 of the hollow fiber membranes 31 in the outermost layer wider than the winding interval P2 of the hollow fiber membranes 31 in the other layers, a circulation path 16 having the same width as a winding interval P1 in which blood can circulate is formed in the outermost layer. The circulation path 16 extends from the distal end toward the proximal end of the housing 10. As a result, a circulation direction of the blood flowing between the inner peripheral surface of the housing 10 and the hollow fiber membrane layer 30 is defined so as to pass through the circulation path 16 to be directed from the distal end side toward the proximal end side of the housing 10. Therefore, the oxygenator 100 can form the flow circulating in the circulation path 16 to flow from the distal end side toward the proximal end side of the housing 10 in the blood flowing through the outermost layer of the hollow fiber membrane layer 30 in the blood chamber 40 of the housing 10. Therefore, in the oxygenator 100, the flow velocity of the blood flowing through the outermost layer of the hollow fiber membrane layer 30 is increased, and furthermore, the blood flow can be uniformized, formation of a stagnation portion in the blood chamber 40 is suppressed, and antithrombogenicity is improved.

In the hollow fiber membrane layer 30, when the winding interval P1 of the hollow fiber membranes 31 in the outermost layer is 1.5 mm or larger, it is easy to form the circulation direction of the blood from the distal end side to the proximal end side of the housing 10 while suppressing the formation of the stagnation portion of the blood.

When the hollow fiber membrane layer 30 is disposed so as to abut the inner peripheral surface of the housing 10 (the inner peripheral surface of the outer cylinder 11), this can more effectively function as the circulation path 16 formed in the hollow fiber membrane 31 of the outermost layer.

The hollow fiber membrane layer 30 has a stacked structure obtained by winding the hollow fiber membranes 31 around the core 20 in multiple layers, and thus can be formed by, for example, winding one or a plurality of bundled hollow fiber membranes 31 with respect to the core 20 in a spiral shape so as to be inclined with respect to the central axis C of the core 20 and substantially parallel to the axis of the core 20. The hollow fiber membrane 31 is formed by forming a large number of hollow fibers having a gas exchange function into a tubular shape.

A forming material of the hollow fiber membrane 31 is not particularly limited as long as the gas exchange with the blood can be performed, and for example, a hydrophobic polymer material such as polypropylene, polyethylene, polysulfone, polyacrylonitrile, polytetrafluoroethylene, or polymethylpentene can be used.

Next, the flow of the blood in the oxygenator 100 will be described.

In the oxygenator 100, the blood circulates in the blood flow path 50 from the blood inflow port 14 to the blood outflow port 15 through the guide portion 14a and the blood chamber 40 in this order.

The blood that flows in from the blood inflow port 14 flows through the guide portion 14a and is guided to the blood chamber 40.

The blood guided to the blood chamber 40 circulates between the inner peripheral surface of the outer cylinder 11 and the outermost layer of the hollow fiber membrane layer 30 and in the hollow fiber membrane layer 30 to expand to flow from the distal end side toward the proximal end side of the housing 10. When passing through the hollow fiber membrane layer 30, the blood exchanges gas with oxygen in the gas exchange unit 32 while moving through the gap of the hollow fiber membranes 31.

The blood flowing between the inner peripheral surface of the outer cylinder 11 and the surface of the outermost layer of the hollow fiber membrane layer 30 passes through the circulation path 16 formed by the hollow fiber membrane 31 wound in the outermost layer of the hollow fiber membrane layer 30 to flow from the distal end toward the proximal end of the housing 10 in the longitudinal direction of the housing 10 in the outermost layer of the hollow fiber membrane layer 30. Therefore, the formation of the stagnation portion of the blood in the outermost layer of the hollow fiber membrane layer 30 is suppressed, and the thrombus is less likely to be formed.

The blood subjected to the gas exchange flows out of the oxygenator 100 from the blood outflow port 15 communicating with the blood chamber 40 and returns to the human body via the extracorporeal circulation circuit.

Next, a manufacturing method of the oxygenator 100 will be described.

First, the core 20 is set in a coupling member connected to a motor of a manufacturing apparatus, and the hollow fiber membrane 31 is spirally wound around the outer periphery of the core 20 while the core 20 is rotated.

At that time, in the hollow fiber membrane layer 30, it is densely wound as for the layers before the outermost layer, and when the outermost layer is formed, the winding interval P1 of the hollow fiber membranes 31 is made wider than the winding interval P2 of the other layers. As a result, the circulation path 16 is formed in the outermost layer of the hollow fiber membrane layer 30 by the interval between the hollow fiber membranes 31.

The number of hollow fiber membranes 31 discharged by the manufacturing apparatus is not particularly limited and may be one or plural (about two to six), and when the number is one, the winding interval of the hollow fiber membranes 31 when winding can be easily adjusted. When a plurality of hollow fiber membranes 31 is wound to reach the outermost layer, and one hollow fiber membrane 31 is wound to form the outermost layer, a manufacturing time of the hollow fiber membrane layer 30 can be shortened, and the production efficiency is increased. In a case where the winding interval of the hollow fiber membranes 31 in the outermost layer is controlled (for example, controlled to the winding interval of 1.5 mm or larger), this can be implemented by appropriately adjusting the number of rotations or the like of winding by a control device and controlling driving of the control device.

When the winding of the hollow fiber membrane 31 is finished, the outer cylinder 11 is attached, both ends of the hollow fiber membrane layer 30 are fixed with urethane, and then both ends are cut. By this process, the end of the hollow fiber membrane layer 30 is exposed, so that oxygen can enter.

Then, the oxygenator 100 is obtained by attaching the distal end and the proximal end of the outer cylinder 11, the lid 13 and the like.

### [Second Embodiment]

Next, an oxygenator according to a second embodiment of the present invention will be described. Note that, in the following description of the embodiment, differences from the above-described embodiment will be mainly described, and components having functions equivalent to those of the first embodiment will be denoted by the same or related reference numerals and will not be described in detail. The configuration, the members, the method of use and the like may be similar to those in the first embodiment.

An oxygenator 100A of the second embodiment has a configuration in which two layers of hollow fiber membrane layers 30 are provided, gas exchange is performed in one layer, and heat exchange is performed in the other layer. Therefore, the hollow fiber membrane layer 30 of the oxygenator 100A includes a gas exchange unit 32 and a heat exchange unit 33.

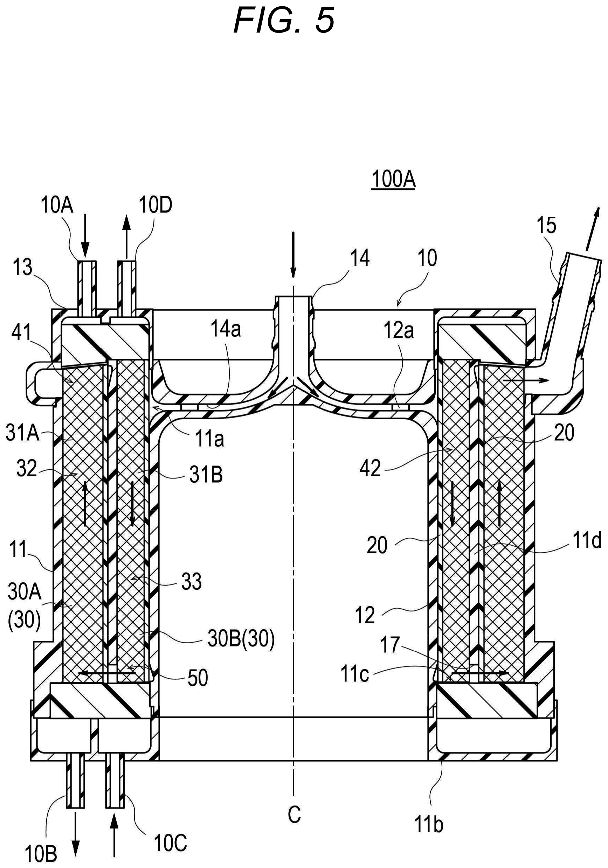

As illustrated in Fig. 5, the oxygenator 100A includes a housing 10, a core 20, the hollow fiber membrane layer 30, a first blood chamber 41, a second blood chamber 42, and a blood flow path 50. A blood inflow port 14 communicates with the second blood chamber 42 via a guide portion 14a. A blood outflow port 15 communicates with the first blood chamber 41 and is formed on a distal end side of an outer cylinder 11 on a downstream side in a blood circulation direction of the blood flow path 50.

The outer cylinder 11 of the housing 10 includes a partition wall 11d that separates a hollow fiber membrane layer 30A (first hollow fiber membrane layer 30A) for gas exchange and a hollow fiber membrane layer 30 (second hollow fiber membrane layer 30B) for heat exchange from each other in an accommodation space 11a that accommodates both of them.

The partition wall 11d is formed over an entire circumference of the outer cylinder 11 in a circumferential direction, and separates the accommodation space 11a into two spaces. Similarly to the inner cylinder 12, the partition wall 11d functions as an annular inner peripheral portion of the housing 10.

On a downstream side in the blood circulation direction of the partition wall 11d, a communication hole 17 that communicates the two spaces separated by the partition wall 11d is formed. The communication hole 17 allows the blood circulating in the second blood chamber 42 to circulate in the first blood chamber 41.

In addition to a gas inflow port 10A and a gas outflow port 10B, the housing 10 of the oxygenator 100A includes a heat medium inflow port 10C that allows a heat medium to flow into the heat exchange unit 33 and a heat medium outflow port 10D that allows the heat medium subjected to the heat exchange to flow out from the heat exchange unit 33. As a result, in the oxygenator 100A, the blood flowing in from the blood inflow port 14 is subjected to the gas exchange when passing through a hollow fiber membrane 31A of the first hollow fiber membrane layer 30A when circulating in the gas exchange unit 32, and is subjected to the heat exchange when passing through a hollow fiber membrane 31B of the second hollow fiber membrane layer 30B when circulating in the heat exchange unit 33. Note that, as illustrated in Fig. 5, the second hollow fiber membrane layer 30B accommodated in the heat exchange unit 33 may be disposed so as to be provided in an entire second blood chamber 42 corresponding to the heat exchange unit 33, or may be disposed so as to form a partial space. That is, the second hollow fiber membrane layer 30B can be appropriately changed according to specifications of the heat exchange unit 33.

From the viewpoint of improving efficiency of the gas exchange in the blood, the gas inflow port 10A is preferably disposed at a position (distal end side of the outer cylinder 11 on a downstream side in the circulation direction of the blood) at which the gas can flow into the first blood chamber 41 opposed to the circulation direction of the blood circulating in the housing 10. The gas outflow port 10B is preferably disposed at a position substantially opposite to the gas inflow port 10A in the longitudinal direction (on the proximal end side of the outer cylinder 11 on an upstream side in the circulation direction of the blood). Note that, the arranging positions of the gas inflow port 10A and the gas outflow port 10B are not limited to the positions illustrated in Fig. 5 as long as they are positions at which heat exchange of blood is possible in the first blood chamber 41.

From the viewpoint of improving efficiency of the heat exchange in the blood, the heat medium inflow port 10C is preferably disposed at a position (proximal end side of the outer cylinder 11 on a downstream side in the circulation direction of the blood) at which the heat medium can flow into the second blood chamber 42 opposed to the circulation direction of the blood circulating in the housing 10. The heat medium outflow port 10D is preferably disposed at a position substantially opposite to the heat medium inflow port 10C in the longitudinal direction (on the distal end side of the outer cylinder 11 on an upstream side in the circulation direction of the blood). Note that, the arranging positions of the heat medium inflow port 10C and the heat medium outflow port 10D are not limited to the positions illustrated in Fig. 5 as long as they are positions at which heat exchange of blood is possible in the second blood chamber 42.

The oxygenator 100A includes the circulation path 16 formed by the winding interval P1 of the hollow fiber membranes 31 between the hollow fiber membrane 31A in the outermost layer of the first hollow fiber membrane layer 30A accommodated in the first blood chamber 41 and the inner peripheral surface of the outer cylinder 11. The oxygenator 100A includes the circulation path 16 formed by the winding interval P1 of the hollow fiber membranes 31 between the hollow fiber membrane 31B in the outermost layer of the second hollow fiber membrane layer 30B accommodated in the second blood chamber 42 and the inner peripheral surface of the partition wall 11d.

Next, the flow of the blood in the oxygenator 100A will be described.

In the oxygenator 100A, the blood circulates in the blood flow path 50 from the blood inflow port 14 to the blood outflow port 15 through the guide portion 14a, the second blood chamber 42, and the first blood chamber 41 in this order.

The blood that flows in from the blood inflow port 14 flows through the guide portion 14a and is guided to the second blood chamber 42.

The blood guided to the second blood chamber 42 circulates between the outer peripheral surface of the inner cylinder 12 and the innermost layer of the second hollow fiber membrane layer 30B and in the second hollow fiber membrane layer 30B to expand to flow from the distal end side toward the proximal end side of the housing 10. When passing through the second hollow fiber membrane layer 30B, the blood exchanges heat in the heat exchange unit 33 while moving through the gap of the hollow fiber membranes 31B.

In the oxygenator 100A, the blood flowing between the inner peripheral surface of the partition wall 11d and the surface of the outermost layer of the second hollow fiber membrane layer 30B in the blood flowing through the second blood chamber 42 passes through the circulation path 16 formed of the hollow fiber membrane 31B to flow in the longitudinal direction of the housing 10 from the distal end toward the proximal end of the housing 10 in the outermost layer of the second hollow fiber membrane layer 30B. Therefore, in the oxygenator 100A, the formation of the stagnation portion of the blood in the outermost layer of the second hollow fiber membrane layer 30B is suppressed, and the thrombus is less likely to be formed.

The blood that passes through the second blood chamber 42 flows into the first blood chamber 41 through the communication hole 17.

The blood that flows into the first blood chamber 41 circulates between the inner peripheral surface of the outer cylinder 11 and the outermost layer of the first hollow fiber membrane layer 30A and in the first hollow fiber membrane layer 30A to expand to flow from the proximal end side toward the distal end side of the housing 10. When passing through the first hollow fiber membrane layer 30A, the blood exchanges gas with oxygen in the gas exchange unit 32 while moving through the gap of the hollow fiber membranes 31A.

In the oxygenator 100A, the blood flowing between the inner peripheral surface of the outer cylinder 11 and the surface of the outermost layer of the first hollow fiber membrane layer 30A in the blood flowing through the first blood chamber 41 passes through the circulation path 16 formed of the hollow fiber membrane 31A to flow in the longitudinal direction of the housing 10 from the proximal end toward the distal end of the housing 10 in the outermost layer of the first hollow fiber membrane layer 30A. Therefore, in the oxygenator 100A, as in the first embodiment, the formation of the stagnation portion of the blood in the outermost layer of the hollow fiber membrane layer 30 is suppressed, and the thrombus is less likely to be formed.

In this manner, in the oxygenator 100A, the blood flowing in from the blood inflow port 14 flows through the second blood chamber 42 in the longitudinal direction from the distal end side to the proximal end side of the housing 10, flows into the first blood chamber 41 through the communication hole 17, then flows in the longitudinal direction from the proximal end side to the distal end side of the housing 10, and flows out from the blood outflow port 15.

The blood subjected to the gas exchange flows out of the oxygenator 100A from the blood outflow port 15 communicating with the first blood chamber 41 and returns to the human body via the extracorporeal circulation circuit.

### [Operational Effects]

As described above, the oxygenator 100 according to the present embodiment is the oxygenator including the housing 10 having a cylindrical shape and including the blood inflow port 14 and the blood outflow port 15, and the hollow fiber membrane layer 30 having the stacked structure obtained by winding the hollow fiber membranes 31 accommodated in the housing 10, the oxygenator in which the blood flowing in from the blood inflow port 14 flows from one end toward the other end in the longitudinal direction of the housing 10 and flows out from the blood outflow port 15, in which, in the hollow fiber membrane layer 30, the winding interval P1 of the hollow fiber membranes wound in the outermost layer in the stacked structure is wider than the winding interval P2 of the hollow fiber membranes in the other layers.

In the hollow fiber membrane layer 30 of the oxygenator 100, the hollow fiber membranes 31 can be sparsely wound only in the outermost layer in the stacked structure as compared to the other layers.

In the hollow fiber membrane layer 30 of the oxygenator 100, the number of windings of the hollow fiber membranes 31 in the outermost layer in the stacked structure may be smaller than the number of windings of the hollow fiber membranes 31 in the other layers.

In the hollow fiber membrane layer 30 of the oxygenator 100, the winding interval of the hollow fiber membranes 31 in the stacked structure is discontinuous, and an interval difference between the winding interval P1 of the hollow fiber membranes 31 in the outermost layer and the winding interval P2 of the hollow fiber membranes 31 in the other layers may be made larger than the interval difference between the winding intervals P2 between any other layers.

With such a configuration, in the oxygenator 100, the winding interval P1 of the hollow fiber membranes 31 of the outermost layer is wider than the winding interval P2 of the hollow fiber membranes 31 of the other layers, and the circulation path 16 in which blood can circulate extending from the distal end toward the proximal end of the housing 10 with the same width as the winding interval P1 can be formed in the outermost layer. As a result, the oxygenator 100 can form the flow in the longitudinal direction of the housing 10 circulating in the circulation path 16 in the blood flowing through the outermost layer of the hollow fiber membrane layer 30 in the blood chamber 40 of the housing 10. Therefore, in the oxygenator 100, the flow velocity of the blood flowing through the outermost layer of the hollow fiber membrane layer 30 is increased, and furthermore, the blood flow can be uniformized, and as a result, formation of a stagnation portion in the blood chamber 40 is suppressed, and antithrombogenicity is improved.

In the hollow fiber membrane layer 30 of the oxygenator 100, the winding interval in the outermost layer in the stacked structure may be made 1.5 mm or larger.

With such a configuration, in the oxygenator 100, the circulation path 16 in which blood can circulate extending from the distal end toward the proximal end of the housing 10 is formed in the outermost layer with a flow path width with which the flow becomes smooth while suppressing the formation of the stagnation portion of blood. Therefore, in the oxygenator 100, the flow velocity of the blood flowing between the housing 10 and the outermost layer of the hollow fiber membrane layer 30 is increased, formation of a stagnation portion in the blood chamber 40 is suppressed, and antithrombogenicity is improved.

The present application is based on Japanese Patent Application No. 2023-164700 filed on September 27, 2023, the disclosed content of which is incorporated herein by reference in its entirety.

### Reference Signs List

10 Housing
10A Gas inflow port
10B Gas outflow port
10C Heat medium inflow port
10D Heat medium outflow port
11 Outer cylinder (11a accommodation space, 11b outer bottom, 11c inner bottom, 11d partition wall)
12 Inner cylinder (12a protrusion)
13 Lid
14 Blood inflow port
15 Blood outflow port
16 Circulation path
17 Communication hole
20 Core
30 Hollow fiber membrane layer
30A First hollow fiber membrane layer
30B Second hollow fiber membrane layer
31, 31A, 31B Hollow fiber membrane
32 Gas exchange unit
33 Heat exchange unit
40 Blood chamber
41 First blood chamber
42 Second blood chamber
50 Blood flow path
100, 100A Oxygenator
C Central axis
P1 Winding interval between hollow fiber membranes in outermost layer of hollow fiber membrane layer
P2 Winding interval between hollow fiber membranes in other layers of hollow fiber membrane layer

## Claims

1. An oxygenator comprising:
a housing having a cylindrical shape and including a blood inflow port and a blood outflow port; and
a hollow fiber membrane layer having a stacked structure obtained by winding hollow fiber membranes, the hollow fiber membrane layer being accommodated in the housing, the oxygenator in which blood flowing in from the blood inflow port flows from one end toward the other end in a longitudinal direction of the housing and flows out from the blood outflow port, wherein, in the hollow fiber membrane layer, a winding interval of the hollow fiber membranes wound in an outermost layer in the stacked structure is wider than a winding interval of the hollow fiber membranes in other layers.

2. The oxygenator according to claim 1, wherein, in the hollow fiber membrane layer, the hollow fiber membranes are sparsely wound only in the outermost layer in the stacked structure as compared with the other layers.

3. The oxygenator according to claim 1, wherein, in the hollow fiber membrane layer, the number of windings of the hollow fiber membranes in the outermost layer in the stacked structure is smaller than the number of windings of the hollow fiber membranes in the other layers.

4. The oxygenator according to claim 1, wherein, in the hollow fiber membrane layer, the winding interval of the hollow fiber membranes in the stacked structure is discontinuous, and an interval difference between the winding interval of the hollow fiber membranes in the outermost layer and the winding interval of the hollow fiber membranes in the other layers is larger than the interval difference between the winding intervals between any other layers.

5. The oxygenator according to any one of claims 1 to 4, wherein, in the hollow fiber membrane layer, a winding interval of an outermost layer in the stacked structure is 1.5 mm or larger.
